# EUROPÄISCHE PATENTSCHRIFT

(11) **EP 1 128 808 B1**
(45) Veröffentlichungstag und Bekanntmachung des Hinweises auf die Patenterteilung: **16.06.2004**
(21) Anmeldenummer: 99955924.8
(22) Anmeldetag: 30.10.1999
(51) Int. Cl.: A61K 7/50

(54) **VERWENDUNG VON KATIONAKTIVEN MISCHUNGEN**
UTILIZATION OF CATION-ACTIVE MIXTURES
UTILISATION DE MELANGES CATIONIQUES

(30) Priorität: 09.11.1998 DE 19851429
(43) Veröffentlichungstag der Anmeldung: 05.09.2001
(73) Patentinhaber: Cognis Deutschland GmbH & Co. KG, 40589 Düsseldorf (DE)
(72) Erfinder: JACKWERTH, Bettina, D-40764 Langenfeld (DE); GASSENMEIER, Thomas, D-40229 Düsseldorf (DE); AMELA CONESA, Cristina, E-08042 Barcelona (ES); PRAT QUERALT, Esther, E-08328 Alella (ES)
(86) Internationale Anmeldenummer: PCT/EP1999/008286
(87) Internationale Veröffentlichungsnummer: WO 2000/027354

(56) Entgegenhaltungen:
- DE-A- 19 724 868
- DE-A- 19 805 703
- DE-C- 19 539 845
- DE-C- 19 539 846
- DE-C- 19 539 876

## Beschreibung

### Gebiet der Erfindung

Die Erfindung befindet sich auf dem Gebiet kationischer Tenside und betrifft die Verwendung von ölhaltigen kosmetischen Zubereitungen mit einem Gehalt an Esterquats, Fettalkoholen und Fettalkoholpolyglycolethern.

### Stand der Technik

Kationische Tenside vom Typ der Esterquats werden in der Kosmetik schon seit einiger Zeit für die Haaravivage eingesetzt. Wegen ihrer guten sensorischen Eigenschaften finden diese Stoffe auch zunehmend Eingang in die Hautkosmetik. Kosmetische Emulsionen, die Esterquats nach dem Stand der Technik als Avivagemittel oder kationische Emulgatoren enthalten, sind jedoch in anwendungstechnischer Hinsicht nicht völlig befriedigend. So wird von Verbrauchern beispielsweise bemängelt, daß die Mittel einen öligen Rückstand hinterlassen, sich nicht rasch genug verteilen und schneller einziehen sollten.

In diesem Zusammenhang sei auf die Deutsche Patentschrift DE-C1 4308794 (Henkel) hingewiesen, aus der ein Verfahren zur Herstellung fester Esterquats bekannt ist, bei dem man die Quaternierung von Triethanolaminestern in Gegenwart von geeigneten Dispergatoren, vorzugsweise Fettalkoholen, durchführt; diese Zubereitungen werden als Konditioniermittel für die Haarbehandlung eingesetzt. In der DE-C1 4335782 (Henkel) wird ferner vorgeschlagen, die Quaternierung von Triethanolaminfettsäureestern in Gegenwart von Polyolen, beispielsweise Glycerin, Ethylenglycol, Partialglyceriden, nichtionischen Tensiden und dergleichen durchzuführen, um die Verwendung von Isopropylalkohol als brennbarem Lösemittel zu vermeiden.

Die Aufgabe der vorliegenden Erfindung hat folglich darin bestanden, ölhaltige kosmetische Zubereitungen mit einem Gehalt an kationischen Tensiden vom Esterquat-Typ zur Verfügung zu stellen, die sich durch verbesserte sensorische Eigenschaften und insbesondere durch rasches Verteilen und rückstandsfreies, schnelles Einziehen auszeichnen.

### Beschreibung der Erfindung

Gegenstand der Erfindung ist die Verwendung von kationaktiven Mischungen, enthaltend
(a) Esterquats,
(b) Ölkörper,
(c) Fettalkohole und
(d) Fettalkoholpolyglycolether
zur Herstellung von Hautreinigungs- und -pflegemitteln.

Überraschenderweise wurde gefunden, daß ölhaltige Hautreinigungs- und -pflegemittel, vorzugsweise in Emulsionsform, die Esterquats zusammen mit Fettalkoholen und Fettalkoholpolyglycolethem, vorzugsweise Mischungen von Cetearylalkohol und Cetearylethoxylaten, enthalten, der Haut einen besonders angenehmen Griff verleihen, keine Rückstände hinterlassen sowie schnell spreiten und rasch einziehen.

### Esterquats

Unter der Bezeichnung "Esterquats" werden im allgemeinen quaternierte Fettsäuretriethanolaminestersalze verstanden. Es handelt sich dabei um bekannte Stoffe, die man nach den einschlägigen Methoden der präparativen organischen Chemie erhalten kann. In diesem Zusammenhang sei auf die Internationale Patentanmeldung WO 91/01295 (Henkel) verwiesen, nach der man Triethanolamin in Gegenwart von unterphosphoriger Säure mit Fettsäuren partiell verestert, Luft durchleitet und anschließend mit Dimethylsulfat oder Ethylenoxid quatemiert. Übersichten zu diesem Thema sind beispielsweise von R.Puchta et al. **in Tens.Surf.Det., 30, 186 (1993)**, M.Brock in **Tens.Surf.Det. 30, 394** (1993), R.Lagerman et al. in **J.Am.Oil.Chem.Soc., 71, 97 (1994)** sowie I.Shapiro in **Cosm.Toil. 109, 77 (1994)** erschienen.

Die die Komponente (a) bildenden Esterquats folgen beispielsweise der Formel (I), 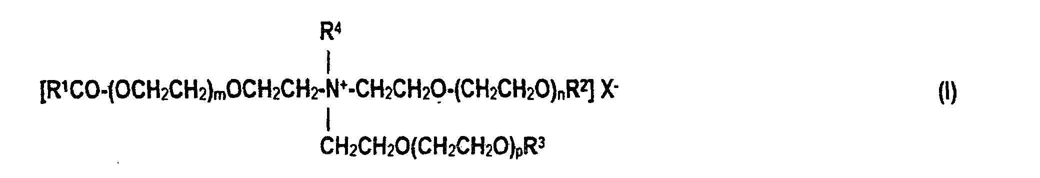 in der R¹CO für einen Acylrest mit 6 bis 22, vorzugsweise 12 bis 18 Kohlenstoffatomen, R² und R3 unabhängig voneinander für Wasserstoff oder R¹CO, R⁴ für einen Alkylrest mit 1 bis 4 Kohlenstoffatomen oder eine (CH₂CH₂O)_{q}H-Gruppe, m, n und p in Summe für 0 oder Zahlen von 1 bis 12, q für Zahlen von 1 bis 12 und X für Halogenid, Alkylsulfat oder Alkylphosphat steht. Zur Herstellung der quaternierten Ester können die Fettsäuren, wie beispielsweise Palmfettsäure, Kokosfettsäure oder Talgfettsäure, und das Triethanolamin im molaren Verhältnis von 1, 1 : 1 bis 3 : 1 eingesetzt werden. Im Hinblick auf die anwendungstechnischen Eigenschaften der Esterquats hat sich ein Einsatzverhältnis von 1,2 : 1 bis 2,2 :1, vorzugsweise 1,5 : 1 bis 1,9 : 1 als besonders vorteilhaft erwiesen. Die bevorzugten Esterquats stellen technische Mischungen von Mono-, Di- und Triestern mit einem durchschnittlichen Veresterungsgrad von 1,5 bis 1,9 dar und leiten sich von technischer C_{12/18}-Palmfettsäure (lodzahl 0 bis 40) ab. Aus anwendungstechnischer Sicht haben sich quaternierte Fettsäuretriethanolaminestersalze der Formel (I) als besonders vorteilhaft erwiesen, in der R¹CO für einen Palmacylrest mit 12 bis 18 Kohlenstoffatomen, R² für R¹CO, R3 für Wasserstoff, R⁴ für eine Methylgruppe, m, n und p für 0 und X für Methylsulfat steht.

Als weitere Gruppe geeigneter Esterquats kommen ferner quaternierte Estersalze der genannten Fettsäuren mit Diethanolalkylaminen der Formel (II) in Betracht. 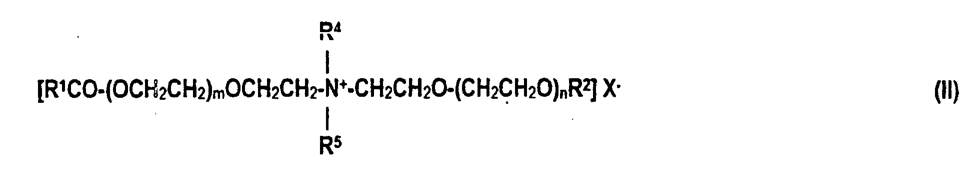 in der R¹CO für einen Acylrest mit 6 bis 22, vorzugsweise 12 bis 18 Kohlenstoffatomen, R² für Wasserstoff oder R¹CO, R⁴ und R⁵ unabhängig voneinander für Alkylreste mit 1 bis 4 Kohlenstoffatomen, m und n in Summe für 0 oder Zahlen von 1 bis 12 und X für Halogenid, Alkylsulfat oder Alkylphosphat steht. Als dritte Gruppe geeigneter Esterquats sind schließlich die quaternierten Estersalze der genannten Fettsäuren mit 1,2-Dihydroxypropyldialkylaminen der Formel (III) zu nennen, 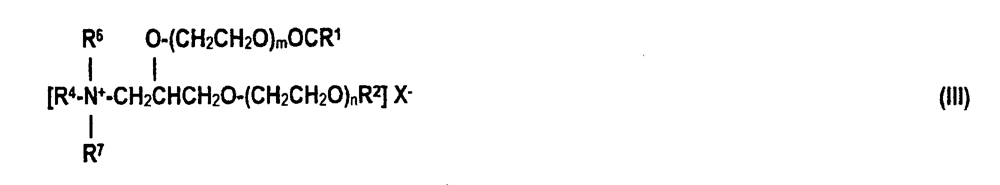 in der R¹CO für einen Acylrest mit 6 bis 22, vorzugsweise 12 bis 18 Kohlenstoffatomen, R² für Wasserstoff oder R¹CO, R⁴, R⁶ und R⁷ unabhängig voneinander für Alkylreste mit 1 bis 4 Kohlenstoffatomen, m und n in Summe für 0 oder Zahlen von 1 bis 12 und X für Halogenid, Alkylsulfat oder Alkylphosphat steht. Hinsichtlich der Auswahl des optimalen Veresterungsgrades gelten die für (I) genannten Beispiele auch für die Esterquats der Formeln (II) und (III).

### Ölkörper

Als Ölkörper kommen beispielsweise Guerbetalkohole auf Basis von Fettalkoholen mit 6 bis 18, vorzugsweise 8 bis 10 Kohlenstoffatomen, Ester von linearen C₆-C₂₂-Fettsäuren mit linearen C₆-C₂₂-Fettalkoholen, Ester von verzweigten C₆-C₁₃-Carbonsäuren mit linearen C₆-C₂₂-Fettalkoholen, Ester von linearen C₆-C₂₂-Fettsäuren mit verzweigten Alkoholen, insbesondere 2-Ethylhexanol, Ester von Hydroxycarbonsäuren mit linearen oder verzweigten C₆-C₂₂-Fettalkoholen, insbesondere Dioctyl Malate, Ester von linearen und/oder verzweigten Fettsäuren mit mehrwertigen Alkoholen (wie z.B. Propylenglycol, Dimerdiol oder Trimertriol) und/oder Guerbetalkoholen, Triglyceride auf Basis C₆-G₁₀-Fettsäuren, flüssige Mono-/Di-/Triglyceridmischungen auf Basis von C₆-C₁₈-Fettsäuren, Ester von C₆-C₂₂-Fettalkoholen und/oder Guerbetalkoholen mit aromatischen Carbonsäuren. Ester von C₂-C₁₂-Dicarbonsäuren mit linearen oder verzweigten Alkoholen mit 1 bis 22 Kohlenstoffatomen oder Polyolen mit 2 bis 10 Kohlenstoffatomen und 2 bis 6 Hydroxylgruppen, pflanzliche Öle, verzweigte primäre Alkohole, substituierte Cyclohexane, lineare und verzweigte C₆-C₂₂-Fettalko-holcarbonate, Guerbetcarbonate, Ester der Benzoesäure mit linearen und/oder verzweigten C₆-C₂₂-Alkoholen (z.B. Finsolv® TN), lineare oder verzweigte, symmetrische oder unsymmetrische Dialkylether mit 6 bis 22 Kohlenstoffatomen pro Alkylgruppe, Ringöffnungsprodukte von epoxidierten Fettsäureestern mit Polyolen, Siliconöle und/oder aliphatische bzw. naphthenische Kohlenwasserstoffe in Betracht.

### Fettalkohole und Fettalkoholpolyglycolether

Unter Fettalkoholen werden primäre, vorzugsweise langkettige und linear aufgebaute Alkohole verstanden, die üblicherweise der Formel (IV) folgen,

R⁸OH

in der R8 für einen linearen oder verzweigten Alkyl- und/oder Alkenylrest mit 6 bis 22 Kohlenstoffatomen steht. Typische Beispiele sind Capronalkohol, Caprylalkohol, Caprinalkohol, Laurylalkohol, Myristylalkohol, Cetylalkohol, Stearylalkohol, Oleylalkohol, Erucylalkohol, Behenylalkohol sowie deren technischen Gemische, wie sie bei der Druckspaltung natürlicher Triglyceride anfallen. Die Fettalkoholpolyglycolether folgen vorzugsweise der Formel (V),

R⁹O(CH₂CH₂O)ₙH

in der R⁹ für einen linearen oder verzweigten Alkyl- und/oder Alkenylrest mit 6 bis 22 Kohlenstoffatomen und n für Zahlen von 1 bis 20 steht. Dabei kann es sich um Anlagerungsprodukte von 1 bis 20, vorzugsweise 10 bis 15 Mol Ethylenoxid an die vorgenannten Fettalkohole handeln, wobei die Ethoxylate sowohl eine konventionell breite als auch eingeengte Homologenverteilung aufweisen können. Es hat sich als vorteilhaft erwiesen, wenn Fettalkohole und Polyglycolether den gleichen Fettrest aufweisen. Vorzugsweise wird Cetearylalkohol eingesetzt, eine 1 : 1-Mischung von Cetyl- und Stearylalkohol bzw. Addukte von 1 bis 20 und insbesondere 10 bis 15 Mol Ethylenoxid an Cetearylalkohol. Weiterhin ist es bevorzugt, Mischungen der Komponenten (a), (c) und (d) zu verwenden, die herstellungsbedingt schon als technische Mischungen anfallen. Hierzu werden Alkanolaminfettsäureester in Gegenwart von solchen Mengen Fettalkohol und Fettalkoholpolyglycolether, vorzugsweise Mischungen von Cetearylalkohol und Ceterylpolyglycolethern, mit Alkylierungsmitteln umgesetzt, daß sich ein Gewichtsverhältnis Esterquat : Fettalkohol/Fettalkoholpolyglycolether von 90 : 10 bis 10 : 90 und vorzugsweise 80 : 20 bis 70 : 30 bzw. 20 : 80 bis 30 : 70 ergibt. Die Veresterung und Quaternierung kann dabei in an sich bekannter Weise durchgeführt werden, wie dies beispielsweise ausführlich in den Druckschriften DE-C1 4308794 und DE-C1 4335782 (Henkel) beschrieben wird. Der besondere Vorteil des Einsatzes derartiger Mischungen liegt darin, daß sie sich auch ohne Erwärmung problemlos dispergieren lassen. Überraschenderweise zeigen sich diese auf direktem Wege hergestellten binären Gemischen in der Formulierung der Mischung der Einzelstoffe auch in sensorischer Hinsicht überlegen.

### Kosmetische Zubereitungen

Wie schon eingangs erläutert, stellen die kosmetischen Zubereitungen der vorliegenden Erfindung üblicherweise Emulsionen dar, wobei es sich sowohl um W/O- als auch O/W-Typen handeln kann; auch multiple Emulsionen vom W/O/W- oder O/W/O-Typ kommen in Frage. In einer bevorzugten Ausführungsform können die erfindungsgemäß zu verwendenden Mischungen folgende Zusammensetzung aufweisen:
(a) 0,1 bis 25, vorzugsweise 5 bis 15 Gew.-% Esterquats,
(b) 0,5 bis 90, vorzugsweise 5 bis 50 Gew.-% Ölkörper,
(c) 0,1 bis 75, vorzugsweise 5 bis 50 Gew.-% Fettalkohole und
(d) 0,1 bis 75, vorzugsweise 5 bis 50 Gew.-% Afkoholpolyglycolether
mit der Maßgabe, daß sich die Mengenangaben mit Wasser und gegebenenfalls weiteren Inhaltsstoffen zu 100 Gew.-% ergänzen.

### Gewerbliche Anwendbarkeit

Die unter der erfindungsgemäßen Verwendung der Mischungen erhältlichen Zubereitungen, wie beispielsweise Haarkuren, Haarconditioner, Haarfärbeemulsionen, Körperreinigungs- und -pflegemittel, Sonnenschutzcremes, -lotionen oder -salben sowie Make-ups und andere dekorative Kosmetikprodukte, können ferner als weitere Hilfs- und Zusatzstoffe milde Tenside, Emulgatoren, Überfettungsmittel, Perlglanzwachse, Stabilisatoren, Konsistenzgeber, Verdickungsmittel, Polymere, Siliconverbindungen, biogene Wirkstoffe, Deowirkstoffe, Antischuppenmittel, Filmbildner, Konservierungsmittel, Hydrotrope, Solubilisatoren, UV-Lichtschutzfaktoren, Antioxidantien, Insektenrepellentien, Selbstbräuner, Parfümöle, Farbstoffe und dergleichen enthalten.

Typische Beispiele für geeignete milde, d.h. besonders hautverträgliche **Tenside** sind Fettalkoholpoly-glycolethersulfate, Monoglyceridsulfate, Mono- und/oder Dialkylsulfosuccinate, Fettsäureisethionate, Fettsäuresarcosinate, Fettsäuretauride, Fettsäureglutamate, Ethercarbonsäuren, Alkyloligoglucoside, Fettsäureglucamide, Alkylamidobetaine und/oder Proteinfettsäurekondensate, letztere vorzugsweise auf Basis von Weizenproteinen.

Als Emulgatoren kommen beispielsweise nichtionogene Tenside aus mindestens einer der folgenden Gruppen in Frage:
(1) Anlagerungsprodukte von 2 bis 30 Mol Ethylenoxid und/ oder 0 bis 5 Mol Propylenoxid an lineare Fettalkohole mit 8 bis 22 C-Atomen, an Fettsäuren mit 12 bis 22 C-Atomen und an Alkylphenole mit 8 bis 15 C-Atomen in der Alkylgruppe;
(2) C_{12/18}-Fettsäuremono- und -diester von Anlagerungsprodukten von 1 bis 30 Mol Ethylenoxid an Glycerin;
(3) Glycerinmono- und -diester und Sorbitanmono- und -diester von gesättigten und ungesättigten Fettsäuren mit 6 bis 22 Kohlenstoffatomen und deren Ethylenoxidanlagerungsprodukte;
(4) Alkylmono- und -oligoglycoside mit 8 bis 22 Kohlenstoffatomen im Alkylrest und deren ethoxylierte Analoga;
(5) Anlagerungsprodukte von 15 bis 60 Mol Ethylenoxid an Ricinusöl und/oder gehärtetes Ricinusöl;
(6) Polyol- und insbesondere Polyglycerinester, wie z.B. Polyglycerinpolyricinoleat, Polyglycerinpoly-12-hydroxystearat oder Polyglycerindimerat. Ebenfalls geeignet sind Gemische von Verbindungen aus mehreren dieser Substanzklassen;
(7) Anlagerungsprodukte von 2 bis 15 Mol Ethylenoxid an Ricinusöl und/oder gehärtetes Ricinusöl;
(8) Partialester auf Basis linearer, verzweigter, ungesättigter bzw. gesättigter-C_{6/22}-Fettsäuren, Ricinolsäure sowie 12-Hydroxystearinsäure und Glycerin, Polyglycerin, Pentaerythrit, Dipenta-erythrit, Zuckeralkohole (z.B. Sorbit), Alkylglucoside (z.B. Methylglucosid, Butylglucosid, Lauryl-glucosid) sowie Polyglucoside (z.B. Cellulose);
(9) Mono-, Di- und Trialkylphosphate sowie Mono-, Di- und/oder Tri-PEG-alkylphosphate und deren Salze;
(10) Wollwachsalkohole;
(11) Polysiloxan-Polyalkyl-Polyether-Copolymere bzw. entsprechende Derivate;
(12) Mischester aus Pentaerythrit, Fettsäuren, Citronensäure und Fettalkohol gemäß DE-PS 1165574 und/oder Mischester von Fettsäuren mit 6 bis 22 Kohlenstoffatomen, Methylglucose und Polyolen, vorzugsweise Glycerin oder Polyglycerin sowie
(13) Polyalkylenglycole.

Die Anlagerungsprodukte von Ethylenoxid und/oder von Propylenoxid an Fettalkohole, Fettsäuren, Alkylphenole, Glycerinmono- und -diester sowie Sorbitanmono- und -diester von Fettsäuren oder an Ricinusöl stellen bekannte, im Handel erhältliche Produkte dar. Es handelt sich dabei um Homologengemische, deren mittlerer Alkoxylierungsgrad dem Verhältnis der Stoffmengen von Ethylenoxid und/ oder Propylenoxid und Substrat, mit denen die Anlagerungsreaktion durchgeführt wird, entspricht. C_{12/18}-Fettsäuremono- und -diester von Anlagerungsprodukten von Ethylenoxid an Glycerin sind aus **DE-PS 2024051** als Rückfettungsmittel für kosmetische Zubereitungen bekannt.

C_{8/18}-Alkylmono- und -oligoglycoside, ihre Herstellung und ihre Verwendung sind aus dem Stand der Technik bekannt. Ihre Herstellung erfolgt insbesondere durch Umsetzung von Glucose oder Oligosacchariden mit primären Alkoholen mit 8 bis 18 C-Atomen. Bezüglich des Glycosidrestes gilt, daß sowohl Monoglycoside, bei denen ein cyclischer Zuckerrest glycosidisch an den Fettalkohol gebunden ist, als auch oligomere Glycoside mit einem Oligomerisationsgrad bis vorzugsweise etwa 8 geeignet sind. Der Oligomerisierungsgrad ist dabei ein statistischer Mittelwert, dem eine für solche technischen Produkte übliche Homologenverteilung zugrunde liegt.

Weiterhin können als Emulgatoren zwitterionische Tenside verwendet werden. Als zwitterionische Tenside werden solche oberflächenaktiven Verbindungen bezeichnet, die im Molekül mindestens eine quartäre Ammoniumgruppe und mindestens eine Carboxylat- und eine Sulfonatgruppe tragen. Besonders geeignete zwitterionische Tenside sind die sogenannten Betaine wie die N-Alkyl-N,N-dimethylammoniumglycinate, beispielsweise das Kokosalkyldimethylammoniumglycinat, N-Acylamino-propyl-N,N-dimethylammoniumglycinate, beispielsweise das Kokosacylaminopropyldimethylammoniumglycinat, und 2-Alkyl-3-carboxylmethyl-3-hydroxyethylimidazoline mit jeweils 8 bis 18 C-Atomen in der Alkyl- oder Acylgruppe sowie das Kokosacylaminoethylhydroxyethylcarboxymethylglycinat. Besonders bevorzugt ist das unter der CTFA-Bezeichnung *Cocamidopropyl Betaine* bekannte Fettsäureamid-Derivat. Ebenfalls geeignete Emulgatoren sind ampholytische Tenside. Unter ampholytischen Tensiden werden solche oberflächenaktiven Verbindungen verstanden, die außer einer C_{8/18}-Alkyl- oder -Acylgruppe im Molekül mindestens eine freie Aminogruppe und mindestens eine -COOH- oder -SO₃H-Gruppe enthalten und zur Ausbildung innerer Salze befähigt sind. Beispiele für geeignete ampholytische Tenside sind N-Alkylglycine, N-Alkylpropionsäuren, N-Alkylaminobuttersäuren, N-Alkyliminodipropionsäuren, N-Hydroxyethyl-N-alkylamidopropylglycine, N-Alkyltaurine, N-Alkylsarcosine, 2-Alkylaminopropionsäuren und Alkylaminoessigsäuren mit jeweils etwa 8 bis 18 C-Atomen in der Alkylgruppe. Besonders bevorzugte ampholytische Tenside sind das N-Kokosalkylaminopropionat, das Kokosacylaminoethylaminopropionat und das C_{12/18}-Acylsarcosin. Neben den ampholytischen kommen auch quartäre Emulgatoren in Betracht, wobei solche vom Typ der Esterquats, vorzugsweise methylquaternierte Difettsäuretriethanolaminester-Salze, besonders bevorzugt sind.

Als **Überfettungsmittel** können Substanzen wie beispielsweise Lanolin und Lecithin sowie polyethoxylierte oder acylierte Lanolin- und Lecithinderivate, Polyolfettsäureester, Monoglyceride und Fettsäurealkanolamide verwendet werden, wobei die letzteren gleichzeitig als Schaumstabilisatoren dienen.

Als **Perlglanzwachse** kommen beispielsweise in Frage: Alkylenglycolester, speziell Ethylenglycoldistearat; Fettsäurealkanolamide, speziell Kokosfettsäurediethanolamid; Partialglyceride, speziell Stearinsäuremonoglycerid; Ester von mehrwertigen, gegebenenfalls hydroxysubstituierte Carbonsäuren mit Fettalkoholen mit 6 bis 22 Kohlenstoffatomen, speziell langkettige Ester der Weinsäure; Fettstoffe, wie beispielsweise Fettalkohole, Fettketone, Fettaldehyde, Fettether und Fettcarbonate, die in Summe mindestens 24 Kohlenstoffatome aufweisen, speziell Lauron und Distearylether; Fettsäuren wie Stearinsäure, Hydroxystearinsäure oder Behensäure, Ringöffnungsprodukte von Olefinepoxiden mit 12 bis 22 Kohlenstoffatomen mit Fettalkoholen mit 12 bis 22 Kohlenstoffatomen und/oder Polyolen mit 2 bis 15 Kohlenstoffatomen und 2 bis 10 Hydroxylgruppen sowie deren Mischungen.

Als **Konsistenzgeber** kommen in neben den Fettalkoholen auch Hydroxyfettalkohole mit 12 bis 22 und vorzugsweise 16 bis 18 Kohlenstoffatomen und daneben Partialglyceride, Fettsäuren oder Hydroxyfettsäuren in Betracht. Bevorzugt ist eine Kombination dieser Stoffe mit Alkyloligoglucosiden und/oder Fettsäure-N-methylglucamiden gleicher Kettenlänge und/oder Polyglycerinpoly-12-hydroxystearaten. Geeignete Verdickungsmittel sind beispielsweise Polysaccharide, insbesondere Xanthan-Gum, Guar-Guar, Agar-Agar, Alginate und Tylosen, Carboxymethylcellulose und Hydroxyethylcellulose, ferner höhermolekulare Polyethylenglycolmono- und -diester von Fettsäuren, Polyacrylate, (z.B. Carbopole® von Goodrich oder Synthalene® von Sigma), Polyacrylamide, Polyvinylalkohol und Polyvinylpyrrolidon, Tenside wie beispielsweise ethoxylierte Fettsäureglyceride, Ester von Fettsäuren mit Polyolen wie beispielsweise Pentaerythrit oder Trimethylolpropan, Fettalkoholethoxylate mit eingeengter Homologenverteilung oder Alkyloligoglucoside sowie Elektrolyte wie Kochsalz und Ammoniumchlorid.

Geeignet**e kationische Polymere** sind beispielsweise kationische Cellulosederivate, wie z.B. eine quatemierte Hydroxyethylcellulose, die unter der Bezeichnung Polymer JR 400® von Amerchol erhältlich ist, kationische Stärke, Copolymere von Diallylammoniumsalzen und Acrylamiden, quaternierte Vinylpyrrolidon/Vinyl-imidazol-Polymere, wie z.B. Luviquat® (BASF), Kondensationsprodukte von Polyglycolen und Aminen, quaternierte Kollagenpolypeptide, wie beispielsweise Lauryldimonium hydroxypropyl hydrolyzed collagen (Lamequat®L/Grünau), quatemierte Weizenpolypeptide, Polyethylenimin, kationische Siliconpolymere, wie z.B. Amidomethicone, Copolymere der Adipinsäure und Dimethylaminohydroxypropyldiethylentriamin (Cartaretine®/Sandoz), Copolymere der Acrylsäure mit Dimethyldiallylammoniumchlorid (Merquat® 550/Chemviron), Polyaminopolyamide, wie z.B. beschrieben in der FR-A 2252840 sowie deren vemetzte wasserlöslichen Polymere, kationische Chitinderivate wie beispielsweise quaterniertes Chitosan, gegebenenfalls mikrokristallin verteilt, Kondensationsprodukte aus Dihalogenalkylen, wie z.B. Dibrombutan mit Bisdialkylaminen, wie z.B. Bis-Dimethylamino-1,3-propan, kationischer Guar-Gum, wie z.B. Jaguar® CBS, Jaguar® C-17, Jaguar® C-16 der Firma Celanese, quatemierte Ammoniumsalz-Polymere, wie z.B. Mirapol® A-15, Mirapol® AD-1, Mirapol® AZ-1 der Firma Miranol.

Als **anionische, zwitterionische, amphotere und nichtionische Polymere** kommen beispielsweise Vinylacetat/Crotonsäure-Copolymere, Vinylpyrrolidon/Vinylacrylat-Copolymere, Vinylacetat/Butylmaleat/ Isobornylacrylat-Copolymere , Methylvinylether/Maleinsäureanhydrid-Copolymere und deren Ester, unvernetzte und mit Polyolen vernetzte Polyacrylsäuren, Acrylamidopropyltrimethylammoniumchlorid/ Acrylat-Copolymere, Octylacrylamid/Methylmethacrylat/tert.Butylaminoethylmethacrylat/2-Hydroxypropylmethacrylat-Copolymere, Polyvinylpyrrolidon, Vinylpyrrolidon/Vinylacetat-Copolymere, Vinylpyrroli-don/Dimethylaminoethylmethacrylat/Vinylcaprolactam-Terpolymere sowie gegebenenfalls derivatisierte Celluloseether und Silicone in Frage.

Geeignete **Siliconverbindungen** sind beispielsweise Dimethylpolysiloxane, Methylphenylpolysiloxane, cyclische Silicone sowie amino-, fettsäure-, alkohol-, polyether-, epoxy-, fluor-, glykosid- und/oder alkylmodifizierte Siliconverbindungen, die bei Raumtemperatur sowohl flüssig als auch harzförmig vorliegen können. Eine detaillierte Übersicht über geeignete flüchtige Silicone findet sich zudem von Todd et al. in **Cosm.Toil. 91, 27 (1976)**.

Typische Beispiele für **Fette** sind Glyceride, als **Wachse** kommen u.a. Bienenwachs, Camaubawachs, Candelillawachs, Montanwachs, Paraffinwachs oder Mikrowachse gegebenenfalls in Kombination mit hydrophilen Wachsen, z.B. Cetylstearylalkohol oder Partialglyceriden in Frage. Als **Stabilisatoren** können Metallsalze von Fettsäuren, wie z.B. Magnesium-, Aluminium- und/oder Zinkstearat bzw. -ricinoleat eingesetzt werden.

Unter **biogenen Wirkstoffen** sind beispielsweise Tocopherol, Tocopherolacetat, Tocopherolpalmitat, Ascorbinsaüre, Desoxyribonucleinsäure, Retinol, Bisabolol, Allantoin, Phytantriol, Panthenol, AHA-Säu-ren, Aminosäuren, Ceramide, Pseudoceramide, essentielle Öle, Pflanzenextrakte und Vitaminkomplexe zu verstehen.
Als Deowirkstoffe kommen z.B. Antiperspirantien wie etwa Aluminiumchlorhydate in Frage. Hierbei handelt es sich um farblose, hygroskopische Kristalle, die an der Luft leicht zerfließen und beim Eindampfen wäßriger Aluminiumchloridlösungen anfallen. Aluminiumchlorhydrat wird zur Herstellung von schweißhemmenden und desodorierenden Zubereitungen eingesetzt und wirkt wahrscheinlich über den partiellen Verschluß der Schweißdrüsen durch Eiweiß- und/oder Polysaccharidfällung [vgl. **J.Soc.Cosm.Chem. 24, 281 (1973)]**. Unter der Marke Locron® der Hoechst AG, Frankfurt/FRG, befindet beispielsweise sich ein Aluminiumchlorhydrat im Handel, das der Formel [Al₂(OH)₅Cl]*2,5 H₂O entspricht und dessen Einsatz besonders bevorzugt ist [vgl. **J.Pharm.Pharmacol. 26, 531 (1975)**]. Neben den Chlorhydraten können auch Aluminiumhydroxylactate sowie saure Aluminium/Zirkoniumsalze eingesetzt werden. Als weitere Deowirkstoffe können Esteraseinhibitoren zugesetzt werden. Hierbei handelt es sich vorzugsweise um Trialkylcitrate wie Trimethylcitrat, Tripropylcitrat, Triisopro-pylcitrat, Tributylcitrat und insbesondere Triethylcitrat (Hydagen® CAT, Henkel KGaA, Düssel-dorf/FRG). Die Stoffe inhibieren die Enzymaktivität und reduzieren dadurch die Geruchsbildung. Wahrscheinlich wird dabei durch die Spaltung des Citronensäureesters die freie Säure freigesetzt, die den pH-Wert auf der Haut soweit absenkt, daß dadurch die Enzyme inhibiert werden. Weitere Stoffe, die als Esteraseinhibitoren in Betracht kommen, sind Dicarbonsäuren und deren Ester, wie beispielsweise Glutarsäure, Glutarsäuremonoethylester, Glutarsäurediethylester, Adipinsäure, Adipinsäuremonoethylester, Adipinsäurediethylester, Malonsäure und Malonsäurediethylester, Hydroxycarbnonsäuren und deren Ester wie beispielsweise Citronensäure, Äpfelsäure, Weinsäure oder Weinsäurediethylester. Antibakterielle Wirkstoffe, die die Keimflora beeinflussen und schweißzersetzende Bakterien abtöten bzw. in ihrem Wachstum hemmen, können ebenfalls in den Stiftzubereitungen enthalten sein. Beispiele hierfür sind Chitosan, Phenoxyethanol und Chlorhexidingluconat. Besonders wirkungsvoll hat sich auch 5-Chlor-2-(2,4-dichlorphenoxy)-phenol erwiesen, das unter der Marke Irgasan® von der Ciba-Geigy, Basel/CH vertrieben wird.

Als **Antischuppenmittel** können Climbazol, Octopirox und Zinkpyrethion eingesetzt werden. Gebräuchliche **Filmbildner** sind beispielsweise Chitosan, mikrokristallines Chitosan, quaterniertes Chitosan, Polyvinylpyrrolidon, Vinylpyrrolidon-Vinylacetat-Copolymerisate, Polymere der Acrylsäurereihe, quaternäre Cellulose-Derivate, Kollagen, Hyaluronsäure bzw. deren Salze und ähnliche Verbindungen. Als **Quellmittel** für wäßrige Phasen können Montmorillonite, Clay Mineralstoffe, Pemulen sowie alkylmodifizierte Carbopoltypen (Goodrich) dienen. Weitere geeignete Polymere bzw. Quellmittel können der Übersicht von R.Lochhead in **Cosm.Toil. 108, 95 (1993)** entnommen werden.

Unter **UV-Lichtschutzfaktoren** sind beispielsweise bei Raumtemperatur flüssig oder kristallin vorliegende organische Substanzen (Lichtschutzfilter) zu verstehen, die in der Lage sind, ultraviolette Strahlen zu absorbieren und die aufgenommene Energie in Form iängerwelliger Strahlung, z.B. Wärme wieder abzugeben. UVB-Filter können öllöslich oder wasserlöslich sein. Als öllösliche Substanzen sind z.B. zu nennen:
- 3-Benzylidencampher bzw. 3-Benzylidennorcampher und dessen Derivate, z.B. 3-(4-Methylben-zyliden)campher wie in der **EP-B1 0693471** beschrieben;
- 4-Aminobenzoesäurederivate, vorzugsweise 4-(Dimethylamino)benzoesäure-2-ethylhexylester, 4-(Dimethylamino)benzoesäure-2-octylester und 4-(Dimethylamino)benzoesäureamylester;
- Ester der Zimtsäure, vorzugsweise 4-Methoxyzimtsäure-2-ethylhexylester, 4-Methoxyzimtsäure-propylester, 4-Methoxyzimtsäureisoamylester 2-Cyano-3,3-phenylzimtsäure-2-ethylhexylester (Oc-tocrylene);
- Ester der Salicylsäure, vorzugsweise Salicylsäure-2-ethylhexylester, Salicylsäure-4-isopropylben-zylester, Salicylsäurehomomenthylester;
- Derivate des Benzophenons, vorzugsweise 2-Hydroxy-4-methoxybenzophenon, 2-Hydroxy-4-meth-oxy-4'-methylbenzophenon, 2,2'-Dihydroxy-4-methoxybenzophenon;
- Ester der Benzalmalonsäure, vorzugsweise 4-Methoxybenzmalonsäuredi-2-ethylhexylester;
- Triazinderivate, wie z.B. 2,4,6-Trianilino-(p-carbo-2'-ethyl-1'-hexyloxy)-1,3,5-triazin und Octyl Tria-zon, wie in der EP-A1 0818450 beschrieben;
- Propan-1,3-dione, wie z.B. 1-(4-tert.Butylphenyl)-3-(4'methoxyphenyl)propan-1,3-dion;
- etotricyclo(5.2.1.0)decan-Derivate, wie in der EP-B1 0694521 beschrieben.

Als wasserlösliche Substanzen kommen in Frage:
- 2-Phenylbenzimidazol-5-sulfonsäure und deren Alkali-, Erdalkali-, Ammonium-, Alkylammonium-, Alkanolammonium- und Glucammoniumsalze;
- Sulfonsäurederivate von Benzophenonen, vorzugsweise 2-Hydroxy-4-methoxybenzophenon-5-sul-fonsäure und ihre Salze;
- Sulfonsäurederivate des 3-Benzylidencamphers, wie z.B. 4-(2-Oxo-3-bornylidenmethyl)benzolsulfonsäure und 2-Methyl-5-(2-oxo-3-bornyliden)sulfonsäure und deren Salze.

Als typische UV-A-Filter kommen insbesondere Derivate des Benzoylmethans in Frage, wie beispielsweise 1-(4'-tert.Butylpheny)-3-(4'-methoxyphenyl)propan-1,3-dion, 4-tert.-Butyl-4'methoxydibenzoylmethan (Parsol 1789), oder 1-Phenyl-3-(4'-isopropylphenyl)-propan-1,3-dion. Die UV-A und UV-B-Filter können selbstverständlich auch in Mischungen eingesetzt werden. Neben den genannten löslichen Stoffen kommen für diesen Zweck auch unlösliche Lichtschutzpigmente, nämlich feindisperse Metall-oxide bzw. Salze in Frage, wie, beispielsweise Titandioxid, Zinkoxid, Eisenoxid, Aluminiumoxid, Ceroxid, Zirkoniumoxid, Silicate (Talk), Bariumsulfat und Zinkstearat. Die Partikel sollten dabei einen mittleren Durchmesser von weniger als 100 nm, vorzugsweise zwischen 5 und 50 nm und insbesondere zwischen 15 und 30 nm aufweisen. Sie können eine sphärische Form aufweisen, es können jedoch auch solche Partikel zum Einsatz kommen, die eine ellipsoide oder in sonstiger Weise von der sphärischen Gestalt abweichende Form besitzen. Weitere geeignete UV-Lichtschutzfilter sind der Übersicht von P.Finkel in **SÖFW-Journal 122, 543 (1996)** zu entnehmen.
Neben den beiden vorgenannten Gruppen primärer Lichtschutzstoffe können auch sekundäre Lichtschutzmittel vom Typ der Antioxidantien eingesetzt werden, die die photochemische Reaktionskette unterbrechen, welche ausgelöst wird, wenn UV-Strahlung in die Haut eindringt. Typische Beispiele hierfür sind Aminosäuren (z.B. Glycin, Histidin, Tyrosin, Tryptophan) und deren Derivate, Imidazole (z.B. Urocaninsäure) und deren Derivate, Peptide wie D,L-Carnosin, D-Carnosin, L-Carnosin und deren Derivate (z.B. Anserin), Carotinoide, Carotine (z.B. α-Carotin, β-Carotin, Lycopin) und deren Derivate, Chlorogensäure und deren Derivate, Liponsäure und deren Derivate (z.B. Dihydroliponsäure), Aurothioglucose, Propylthiouracil und andere Thiole (z.B. Thioredoxin, Glutathion, Cystein, Cystin, Cystamin und deren Glycosyl-, N-Acetyl-, Methyl-, Ethyl-, Propyl-, Amyl-, Butyl- und Lauryl-, Palmitoyl-, Oleyl-, γ-Linoleyl-, Cholesteryl- und Glycerylester) sowie deren Salze, Dilaurylthiodipropionat, Distearylthiodipropionat, Thiodipropionsäure und deren Derivate (Ester, Ether, Peptide, Lipide, Nukleotide, Nuk-leoside und Salze) sowie Sulfoximinverbindungen (z.B. Buthioninsulfoximine, Homocysteinsulfoximin, Butioninsulfone, Penta-, Hexa-, Heptathioninsulfoximin) in sehr geringen verträglichen Dosierungen (z.B. pmol bis µmol/kg), ferner (Metall)-Chelatoren (z.B. α-Hydroxyfettsäuren, Palmitinsäure, Phy-tinsäure, Lactoferrin), α-Hydroxysäuren (z.B. Citronensäure, Milchsäure, Apfelsäure), Huminsäure, Gallensäure, Gallenextrakte, Bilirubin, Biliverdin, EDTA, EGTA und deren Derivate, ungesättigte Fett-säuren und deren Derivate (z.B. γ-Linolensäure, Linolsäure. Ölsäure), Folsäure und deren Derivate, Ubichinon und Ubichinol und deren Derivate, Vitamin C und Derivate (z.B. Ascorbylpalmitat, Mg-Ascorbylphosphat, Ascorbylacetat), Tocopherole und Derivate (z.B. Vitamin-E-acetat), Vitamin A und Derivate (Vitamin-A-palmitat) sowie Koniferylbenzoat des Benzoeharzes, Rutinsäure und deren Derivate, α-Glycosylrutin, Ferulasäure, Furfurylidenglucitol, Carnosin, Butylhydroxytoluol, Butylhydroxyanisol, Nordihydroguajakharzsäure, Nordihydroguajaretsäure, Trihydroxybutyrophenon, Harnsäure und deren Derivate, Mannose und deren Derivate, Superoxid-Dismutase, Zink und dessen Derivate (z.B. ZnO, ZnSO₄ Selen und dessen Derivate (z.B. Selen-Methionin), Stilbene und deren Derivate (z.B. Stilbenoxid, trans-Stilbenoxid) und die erfindungsgemäß geeigneten Derivate (Salze, Ester, Ether, Zucker, Nukleotide, Nukleoside, Peptide und Lipide) dieser genannten Wirkstoffe.

Zur Verbesserung des Fließverhaltens können ferner **Hydrotrope**, wie beispielsweise Ethanol isopropylalkohol, oder Polyole eingesetzt werden. Polyole, die hier in Betracht kommen, besitzen vorzugsweise 2 bis 15 Kohlenstoffatome und mindestens zwei Hydroxylgruppen. Typische Beispiele sind
- Glycerin;
- Alkylenglycole, wie beispielsweise Ethylenglycol, Diethylenglycol, Propylenglycol, Butylenglycol, Hexylenglycol sowie Polyethylenglycole mit einem durchschnittlichen Molekulargewicht von 100 bis 1.000 Dalton;
- technische Oligoglyceringemische mit einem Eigenkondensationsgrad von 1,5 bis 10 wie etwa technische Diglyceringemische mit einem Diglyceringehalt von 40 bis 50 Gew.-%;
- Methyolverbindungen, wie insbesondere Trimethylolethan, Trimethylolpropan, Trimethylolbutan, Pentaerythrit und Dipentaerythrit;
- Niedrigalkylglucoside, insbesondere solche mit 1 bis 8 Kohlenstoffen im Alkylrest, wie beispielsweise Methyl- und Butylglucosid;
- Zuckeralkohole mit 5 bis 12 Kohlenstoffatomen, wie beispielsweise Sorbit oder Mannit,
- Zucker mit 5 bis 12 Kohlenstoffatomen, wie beispielsweise Glucose oder Saccharose;
- Aminozucker, wie beispielsweise Glucamin.

Als Konservierungsmittel eignen sich beispielsweise Phenoxyethanol, Formaldehydlösung, Parabene, Pentandiol oder Sorbinsäure sowie die in Anlage 6, Teil A und B der Kosmetikverordnung aufgeführten weiteren Stoffklassen. Als Insekten-Repellentien kommen N,N-Diethyl-m-touluamid, 1,2-Pentandiol oder Insect repellent 3535 in Frage, als Selbstbräuner eignet sich Dihydroxyaceton.

Als **Parfümöle** seien genannt Gemische aus natürlichen und synthetischen Riechstoffen. Natürliche Riechstoffe sind Extrakte von Blüten (Lilie, Lavendel, Rosen, Jasmin, Neroli, Ylang-Ylang), Stengeln und Blättern (Geranium. Patchouli, Petitgrain), Früchten (Anis, Koriander, Kümmel, Wacholder), Fruchtschalen (Bergamotte, Zitrone, Orangen), Wurzeln (Macis, Angelica, Sellerie, Kardamon, Costus, iris, Calmus), Hölzern (Pinien-, Sande)-, Guajak-, Zedern-. Rosenholz), Kräutern und Gräsern (Estragon, Lemongras, Salbei, Thymian), Nadeln und Zweigen (Fichte, Tanne, Kiefer, Latschen), Harzen und Balsamen (Galbanum, Elemi, Benzoe, Myrrhe, Olibanum, Opoponax). Weiterhin kommen tierische Rohstoffe in Frage, wie beispielsweise Zibet und Castoreum. Typische synthetische Riechstoffverbindungen sind Produkte vom Typ der Ester, Ether, Aldehyde, Ketone, Alkohole und Kohlenwasserstoffe. Riechstoffverbindungen vom Typ der Ester sind z.B. Benzylacetat, Phenoxyethylisobutyrat, p-tert.-Butylcyclohexylacetat, Linalylacetat, Dimethylbenzylcarbinylacetat, Phenylethylacetat, Linalylbenzoat, Benzylformiat. Ethylmethylphenylglycinat, Allylcyclohexylpropionat, Styrallylpropionat und Benzylsalicylat. Zu den Ethem zählen beispielsweise Benzylethylether, zu den Aldehyden z.B. die linearen Alkanale mit 8 bis 18 Kohlenstoffatomen, Citral, Citronellal, Citronellyloxyacetaldehyd, Cyclamenaldehyd, Hydroxycitronellal, Lilial und Bourgeonal, zu den Ketonen z.B. die Jonone, ∝-Iso-methylionon und Methylcedrylketon, zu den Alkoholen Anethol, Citronellol, Eugenol, Isoeugenol, Geraniol, Linalool, Phenylethylalkohol und Terpineol, zu den Kohlenwasserstoffen gehören hauptsächlich die Terpene und Balsame. Bevorzugt werden jedoch Mischungen verschiedener Riechstoffe verwendet, die gemeinsam eine ansprechende Duftnote erzeugen. Auch ätherische Öle geringerer Flüchtigkeit, die meist als Aromakomponenten verwendet werden, eignen sich als Parfümöle, z.B. Salbeiöl, Kamillenöl, Nelkenöl, Melissenöl, Minzenöl, Zimtblätteröl, Lindenblütenöl, Wacholderbeerenöl, Vetiveröl, Olibanöl, Galbanumöl, Labolanumöl und Lavandinöl. Vorzugsweise werden Bergamotteöl, Dihydromyrcenol, Lilial, Lyral, Citronellol, Phenylethylalkohol, α-Hexylzimtaldehyd, Geraniol, Benzylaceton, Cyclamenaldehyd, Linalool, Boisambrene Forte, Ambroxan, Indol, Hedione, Sandelice, Citronenöl, Mandarinenöl, Orangenöl, Allylamylglycolat, Cyclovertal, Lavandinöl, Muskateller Salbeiöl, β-Damascone, Geraniumöl Bourbon, Cyclohexylsalicylat, Vertofix Coeur, Iso-E-Super, Fixolide NP, Evernyl, lraldein gamma, Phenylessigsäure, Geranylacetat, Benzylacetat, Rosenoxid, Romilllat, Irotyl und Floramat allein oder in Mischungen, eingesetzt.

Als Farbstoffe können die für kosmetische Zwecke geeigneten und zugelassenen Substanzen verwendet werden, wie sie beispielsweise in der Publikation **"Kosmetische Färbemittel" der Farbstoffkommission der Deutschen Forschungsgemeinschaft, Verlag Chemie, Weinheim, 1984, S.81-106** zusammengestellt sind. Diese Farbstoffe werden üblicherweise in Konzentrationen von 0,001 bis 0,1 Gew.-%, bezogen auf die gesamte Mischung, eingesetzt.

Der Gesamtanteil der Hilfs- und Zusatzstoffe kann 1 bis 50, vorzugsweise 5 bis 40 Gew.-% - bezogen auf die Mittel - betragen. Die Herstellung der Mittel kann durch übliche Kalt- oder Heißprozesse erfolgen; vorzugsweise arbeitet man nach der Phaseninversionstemperatur-Methode.

### Beispiele

**Tabelle 1**

| Kosmetische Zubereitungen (Wasser, Konservierungsmittel ad 100 Gew.-%) | | | | | | | | | | |
|---|---|---|---|---|---|---|---|---|---|---|
| **Zusammensetzung (INCI)** | **1** | **2** | **3** | **4** | **5** | **6** | **7** | **8** | **9** | **10** |
| **Texapon® NSO** Sodium Laureth Sulfate | - | 30,0 | - | - | 25,0- | - | - | - | - | - |
| **Plantacare® 818** Coco Glucosides | - | 10,0 | 30,0 | - | 20,0 | - | - | - | - | - |
| **Plantacare® PS 10** Sodium Laureth Sulfate (and) Coco Glucosides | 22,0 | - | - | 22,0 | - | - | - | - | - | - |
| **Dehyquart® C 4046** Dipalmoyl Methyl Triethanolammonium Methosulfate (and) Cetearyl Alcohol (and) Ceteareth-10 | 1,0 | 1,0 | 1,0 | 1,0 | 1,0 | 1,0 | 1,0 | 1,0 | 1,0 | 1.0 |
| **Dehyton® PK 45** Cocamidopropyl Betaine | 15,0 | 10,0 | 10,0 | 15,0 | 20,0 | - | - | - | - | - |
| **Emulgade® SE** Glyceryl Sterate (and) Ceteareth 12/20 (and) Cetearyl Alcohol (and) Cetyl Palmitate | - | - | - | - | - | 5,0 | 5,0 | - | 5,0 - | - |
| **Eumulgin® B1** Ceteareth-12 | - | - | 15,0 | - | - | - | - | - | - | - |
| **Eumulgin ® HRE 60** PEG 60 Hydrogenated Castor Oil | - | - | - | - | 5,0 | - | - | - | - | - |
| **Lameform® TGI** Polyglyceryl-3 Isostearate | - | - | - | - | - | - | - | 4,0 | - | - |
| **Dehymuls® PGPH** Polyglyceryl-2 Dipolyhydroxystearate | - | - | 3,8 | - | - | - | - | - | - 4.0 | - |
| **Monomuls**® **90-O 18** Glyceryl Oleate | - | - | - | - | - | - | - | 2,0 | - | 2.0 |
| **Cetiol® HE** PEG-7 Glyceryl Cocoate | 2,0 | - | - | 2,0 | 5,0 | - | - | - | - | - |
| **Cetiol® OE** Dicaprylyl Ether | - | - | - | - | - | - | - | 5,0 | - | 5.0 |
| **Cetiol® PGL** Hexyldecanol (and) Hexyldecyl Laurate | - | - | - | - | - | - | - | 10,0 | 3,0 | 10.0 |
| **Cetiol® SN** Cetearyl lsononanoate | - | - | - | - - | - | 3,0 | 3,0 | - | - | - |
| **Cetiol® V** Decyl Oleate | - | - | 5,0 | - | - | 3,0 | 3,0 | - | 3,0 | - |
| **Myritol® 318** Coco Caprylate Caprate | - | - | - | - | - | - | - | 5,0 | - | 5.0 |
| **Bees Wax** | - | - | - | - | - | - | - | 7,0 | - | 7.0 |
| **Nutrilan® Keratin W** Hydrotyzed Keratin | - | - | - | - | - | 40,0 | 60,0 | - | 60,0 | - |
| **Lamesoft® LMG** Glyceryl Laurate (and) Potassium Cocoyl Hydrolyzed Collagen | - | 4,0 | - | - | 2,0 | - | - | - | - | - |
| **Gluadin® WK** Sodium Cocoyl Hydrolyzed Wheat Protein | 2,0 | 2,0 | 4,0 | 2,0 | 5,0 | - | - | 5,0 | **-** | 5.0 |
| **Euperlan® PK 3000 AM** Glycol Distearate (and) Laureth-4 (and) cocamidopropyl Betaine | 5,0 | - | - | 5,0 | - | - | - | - | - | - |
| **Arlypon ®** Laureth-2 | - | - | 1,5 | - | - | - | - | - | - | - |
| Glycerin (86 Gew.-%ig) | - | - | - | - | - | 3.0 | 3,0 | 5,0 | 3.0 | 5.0 |
| (1-5) Schaumbad, (6) Softcreme, (7,9) Feuchtigkeitsemulsion, (8,10) Nachtcreme | | | | | | | | | | |

| **Zusammensetzung (INCI)** | **11** | **12** | **13** | **14** | **15** | **16** | **17** | **18** | **19** | **20** |
|---|---|---|---|---|---|---|---|---|---|---|
| **Dehymuls® PGPH** Polyglyceryl-2 Dipolyhydroxystearate | 2,0 | 3,0 | - | 5,0 | - | - | - | - | - | - |
| **Lameform® TGI** Polyglyceryl-3 Diisostearate | 4,0 | 1,0 | - | - | - | - | - | - | - | - |
| **Emulgade® PL 68/50** Cetearyl Glucoside (and) Cetearyl Alcohol | - | - | - | - | 4,0 | - | - | - | 3,0 | - |
| **Eumulgin®B2** Ceteareth-20 | - | - | - | - | - | - | - | 2,0 | - | - |
| **Tegocare® PS** Polyglyceryl-3 Methylglucose Distearate | - | - | - | - | - | - | 4,0 | - | - | - |
| **Eumulgin VL 75** Polyglyceryl-2 Dipolyhydroxystearate (and) Lauryl Glucoside (and) Glycerin | - | - | - | - | - | 3,5 | - | - | 2,5 | - |
| **Bees Wax** | 3,0 | 2,0 | 5,0 | 2,0 | - | - | - | - | - | - |
| **Cutina® GMS** Glyceryl Stearate | - | - | - | - | - | 2,0 | 4,0 | - | - | 4,0 |
| **Lanette® O** Cetearyl Alcohol | - | - | 2,0 | - | 2,0 | 4,0 | 2,0 | 4,0 | 4,0 | 1,0 |
| **Plantaren® 818** Cocoglycerides | 5,0 | - | 10,0 | - | 8,0 | 6,0 | 6,0 | - | 5,0 | 5,0 |
| **Dehyquart® C 4046** Dipalmoyl Methyl Triethanolammonium Methosulfate (and) Cetearyl Alcohol (and) Ceteareth-10 | 1,0 | 1,0 | 1,0 | 1,0 | 1,0 | 1,0 | 1,0 | 1,0 | 1.0 | 1,0 |
| **Finsolv® TN** C12/15 Alkyl Benzoate | - | 6,0 | - | 2,0 | - | - | 3,0 | - | - | 2,0 |
| **Dioctyl Carbonate** | 5.0 | 4, 0 | 6, | 0 | 6, 0 | 5, 0 | 4,0 | 3,0 | 4.0 | 6,0 |
| **Cetiol® J 600** Oleyl Erucate | 2,0 | - | 3.0 | 5,0 | 4,0 | 3,0 | 3,0 | - | 5.0 | 4,0 |
| **Cetiol® OE** Dicaprylyl Ether | 3,0 | - | - | - | - | 1,0 | - | - | - | - |
| **Mineral Oil** | - | 4,0 | - | 4,0 | - | 2.0 | - | 1,0 | - | - |
| **Cetiol® PGL** Hexadecanol (and) Hexyldecyl Laurate | - | 7,0 | 3,0 | 7,0 | 4,0 | - | - | - | 1,0 | - |
| **Panthenol / Bisabolol** | 1,2 | 1,2 | 1,2 | 1,2 | 1,2 | 1.2 | 1,2 | 1,2 | 1,2 | 1,2 |
| **Copherol® F 1300** Tocopherol / Tocopheyl Acetate | 0,5 | 1,0 | 1,0 | 2,0 | 1,0 | 1,0 | 1,0 | 2,0 | 0,5 | 2.0 |
| **Neo Heliopan® Hydro** Sodium Phenylbenzimidazole Sulfonate | 3,0 | - | - | 3,0 | - | - | 2,0 | - | 2,0 | - |
| **Neo Heliopan® 303** Octocrylene | - | 5,0 | - | - | - | 4,0 | 5,0 | - | - | 10.0 |
| **Neo Heliopan® BB** Benzophenone-3 | 1,5 | - | - | 2,0 | 1,5 | - | - | - | 2,0 | - |
| **Neo Heliopan® E 1000** Isoamyl p-Methoxycinnamate | 5,0 | - | 4,0 | - | 2,0 | 2,0 | 4,0 | 10,0 | - | - |
| **Neo Heliopan® AV** Octyl Methoxycinnamate | 4,0 | - | 4,0 | 3,0 | 2,0 | 3,0 | 4,0 | - | 10,0 | 2,0 |
| **Uvinul® T 150** octyl triazone | 2,0 | 4,0 | 3,0 | 1,0 | 1,0 | 1,0 | 4,0 | 3,0 | 3,0 | 3,0 |
| **Zinc Oxide** | - | 6,0 | 6,0 | - | 4,0 | | - - | - | - | 5,0 |
| **Titanium Dioxide** | - | 2,0 | 2,0 | - | - | - | - | 5,0 | - | - |
| **Glycerin (86 Gew.-%ig)** | 5,0 | 5,0 | 5,0 | 5,0 | 5,0 | 5,0 | 5,0 | 5,0 | 5,0 | 5,0 |
| (11) W/O-Sonnenschutzcreme, (12-14) W/O-Sonnenschutzlotion, (15, 18, 20) O/W-Sonnenschutziotion (16.17.19) O/W-Sonnenschutzcreme | | | | | | | | | | |

## Patentansprüche

1. Verwendung von kationaktiven Mischungen, enthaltend
(a) Esterquats,
(b) Ölkörper,
(c) Fettalkohole und
(d) Fettalkoholpolyglycolether
zur Herstellung von Hautreinigungs- und -pflegemitteln.

2. Verwendung nach Anspruch 1, **dadurch gekennzeichnet, daß** man Esterquats der Formel (I) einsetzt, 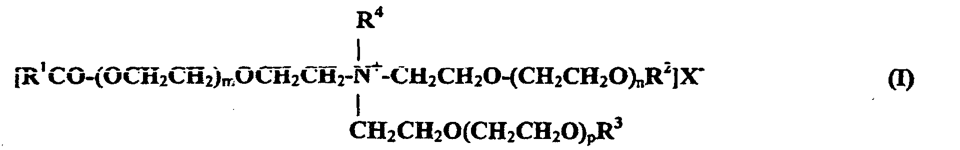 in der R¹CO für einen Acylrest mit 6 bis 22 Kohlenstoffatomen, R² und R³ unabhängig voneinander für Wasserstoff oder R¹CO, R⁴ für einen Alkylrest mit 1 bis 4 Kohlenstoffatomen oder eine (CH₂CH₂O)_{q}H-Gruppe, m, n und p in Summe für 0 oder Zahlen von 1 bis 12, q für Zahlen von 1 bis 12 und X für Halogenid, Alkylsulfat oder Alkylphosphat steht.

3. Verwendung nach Anspruch 1, **dadurch gekennzeichnet, daß** man Esterquats der Formel (II) einsetzt, 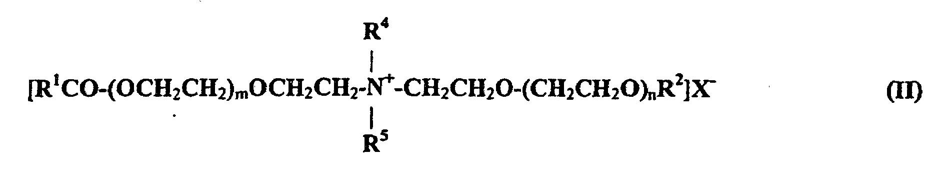 in der R¹CO für einen Acylrest mit 6 bis 22 Kohlenstoffatomen, R² für Wasserstoff oder R¹CO, R⁴ und R⁵ unabhängig voneinander für Alkylreste mit 1 bis 4 Kohlenstoffatomen, m und n in Summe für 0 oder Zahlen von 1 bis 12 und X für Halogenid, Alkylsulfat oder Alkylphosphat steht.

4. Verwendung nach Anspruch 1, **dadurch gekennzeichnet, daß** man Esterquats der Formel (**III**) einsetzt, 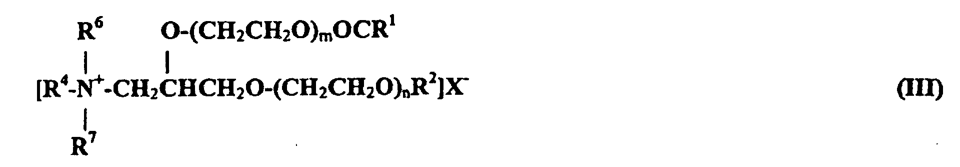 in der R¹CO für einen Acylrest mit 6 bis 22 Kohlenstoffatomen; R² für Wasserstoff oder R¹CO, R⁴, R⁶ und R⁷ unabhängig voneinander für Alkylreste mit 1 bis 4 Kohlenstoffatomen, m und n in Summe für 0 oder Zahlen von 1 bis 12 und X für Halogenid, Alkylsulfat oder Alkylphosphat steht.

5. Verwendung nach mindestens einem der Ansprüche 1 bis 4, **dadurch gekennzeichnet, daß** man Fettalkohole der Formel (IV) einsetzt,
**R**^{**8**}**OH (IV)**
in der R⁸ für einen linearen oder verzweigten Alkyl- und/oder Alkenylrest mit 6 bis 22 Kohlenstoffatomen steht.

6. Verwendung nach mindestens einem der Ansprüche 1 bis 5, **dadurch gekennzeichnet, daß** man Fettalkoholpolyglycolether der Formel (V) einsetzt,
**R**^{**9**}**O(CH**_{**2**}**CH**_{**2**}**O)**_{**n**}**H (V)**
in der R⁹ für einen linearen oder verzweigten Alkyl- und/oder Alkenylrest mit 6 bis 22 Kohlenstoffatomen und n für Zahlen von 1 bis 20 steht.

7. Verwendung nach mindestens einem der Ansprüche 1 bis 6, **dadurch gekennzeichnet, daß** man Mischungen von Esterquats und Fettalkoholen einsetzt, die auf direktem Wege durch Alkylierung von Alkanolaminfettsäureestern in Mischungen der Fettalkohole und Fettalkoholpolyglycolether erhalten werden.

8. Verwendung nach mindestens einem der Ansprüche 1 bis 7, **dadurch gekennzeichnet, daß** man
(a) 0,1 bis 25 Gew.-% Esterquats,
(b) 0,5 bis 90 Gew.-% Ölkörper,
(c) 0,1 bis 75 Gew.-% Fettalkohole und
(d) 0,1 bis 75 Gew.-% Fettalkoholpolyglycolether
mit der Maßgabe einsetzt, daß sich die Mengenangaben mit Wasser und gegebenenfalls weiteren Inhaltsstoffen zu 100 Gew.-% ergänzen.

9. Verwendung nach mindestens einem der Ansprüche 1 bis 8, **dadurch gekennzeichnet, daß** man die Esterquats einerseits und andererseits die Mischungen der Fettalkohole und Fettalkoholpolyglycolether in einem Gewichtsverhältnis von 10 : 90 bis 90 : 10 einsetzt.

## Claims

1. The use of cationic mixtures containing
(a) esterquats,
(b) oil components,
(c) fatty alcohols and
(d) fatty alcohol polyglycol ethers
for the production of skin-cleansing and skin-care products.

2. The use claimed in claim 1, **characterized in that** esterquats corresponding to formula (I): 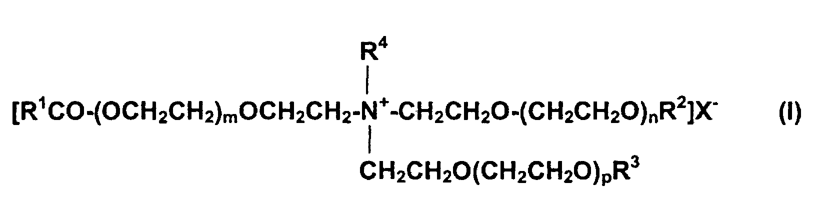 in which R¹CO is an acyl group containing 6 to 22 carbon atoms, R² and R³ independently of one another represent hydrogen or have the same meaning as R¹CO, R⁴ is an alkyl group containing 1 to 4 carbon atoms or a (CH₂CH₂O)_{q}H group, m, n and p together stand for 0 or numbers of 1 to 12, q is a number of 1 to 12 and X is halide, alkyl sulfate or alkyl phosphate, are used.

3. The use claimed in claim 1, **characterized in that** esterquats corresponding to formula (II): 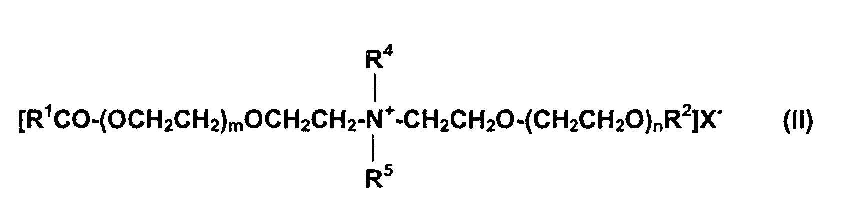 in which R¹CO is an acyl group containing 6 to 22 carbon atoms, R² is hydrogen or has the same meaning as R¹CO, R⁴ and R⁵ independently of one another are alkyl groups containing 1 to 4 carbon atoms, m and n together stand for 0 or numbers of 1 to 12 and X stands for halide, alkyl sulfate or alkyl phosphate, are used

4. The use claimed in claim 1, **characterized in that** esterquats corresponding to formula (III): 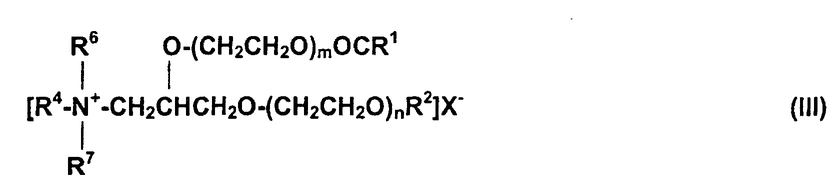 in which R¹CO is an acyl group containing 6 to 22 carbon atoms, R² is hydrogen or has the same meaning as R¹CO, R⁴, R⁶ and R⁷ independently of one another are alkyl groups containing 1 to 4 carbon atoms, m and n together stand for 0 or numbers of 1 to 12 and X stands for halide, alkyl sulfate or alkyl phosphate, are used.

5. The use claimed in at least one of claims 1 to 4, **characterized in that** fatty alcohols corresponding to formula (IV):
R⁸OH (IV)
in which R⁸ is a linear or branched alkyl and/or alkenyl group containing 6 to 22 carbon atoms, are used.

6. The use claimed in at least one of claims 1 to 5, **characterized in that** fatty alcohol polyglycol ethers corresponding to formula (V):
R⁹O(CH₂CH₂O)ₙH (V)
in which R⁹ is a linear or branched alkyl and/or alkenyl group containing 6 to 22 carbon atoms and n is a number of 1 to 20, are used.

7. The use claimed in at least one of claims 1 to 6, **characterized in that** mixtures of esterquats and fatty alcohols directly obtained by alkylation of alkanolamine fatty acid esters in mixtures of the fatty alcohols and fatty alcohol polyglycol ethers are used.

8. The use claimed in at least one of claims 1 to 7, **characterized in that**
(a) 0.1 to 25% by weight of esterquats
(b) 0.5 to 90% by weight of oil components,
(c) 0.1 to 75% by weight of fatty alcohols and
(d) 0.1 to 75% by weight of fatty alcohol polyglycol ethers,
are used, with the proviso that the quantities shown add up to 100% by weight with water and optionally other ingredients.

9. The use claimed in at least one of claims 1 to 8, **characterized in that** the esterquats on the one hand and the mixtures of the fatty alcohols and fatty alcohol polyglycol ethers on the other hand are used in a ratio by weight of 10:90 to 90:10.

## Revendications

1. Utilisation de mélanges à cations actifs contenant
a) des esterquats,
b) des composés huileux,
c) des alcools gras et
d) des éthers d'alcool gras et de polyglycol
en vue de la production de produits de nettoyage de la peau et de produits de soins de la peau.

2. Utilisation selon la revendication 1,
**caractérisée en ce qu'**
on met en oeuvre des esterquats de formule (I) 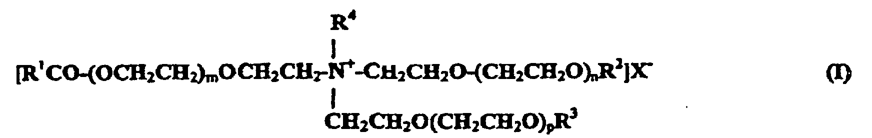 dans laquelle R¹CO représente un reste acyle ayant de 6 à 22 atomes de carbone, R² et R³ indépendamment l'un de l'autre représentent de l'hydrogène ou R¹CO, R⁴ représente un reste alkyle ayant de 1 à 4 atomes de carbone ou un groupe (CH₂CH₂O)_{q}H, m, n et p globalement représentent 0 ou des nombres allant de 1 à 12, q vaut 1 à 12 et X représente un halogénure, un alkylsulfate ou un alkylphosphate.

3. Utilisation selon la revendication 1,
**caractérisée en ce qu'**
on met en oeuvre des esterquats de formule (II) 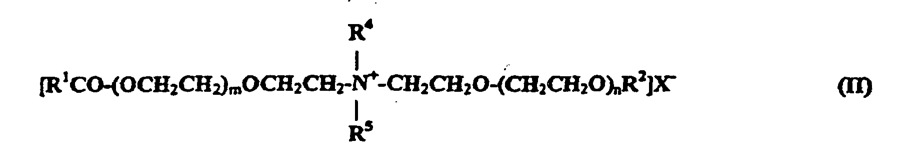 dans laquelle R¹CO représente un reste acyle ayant de 6 à 22 atomes de carbone, R² représente de l'hydrogène ou R¹CO, R⁴ et R⁵ indépendamment l'un de l'autre, représentent des restes alkyle ayant de 1 à 4 atomes de carbone, m et n globalement représentent 0 ou des nombres allant de 1 à 12, et X représente un halogénure, un alkyl sulfate ou un alkylphosphate.

4. Utilisation selon la revendication 1,
**caractérisée en ce qu'**
on met en oueuvre des esterquats de formule (III) 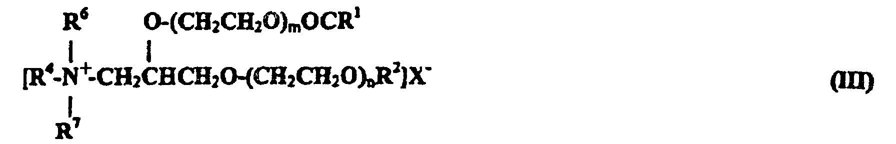 dans laquelle R¹CO représente un reste acyle ayant de 6 à 22 atomes de carbone, R² représente de l'hydrogène ou R¹CO, R⁴, R⁶ et R⁷ indépendamment les uns des autres représentent des restes alkyle ayant de 1 à 4 atomes de carbone, m et n globalement représentent O ou des nombres allant de 1 à 12 et X représente un halogénure, un alkylsulfate ou un alkylphosphate.

5. Utilisation selon au moins l'une des revendications 1 à 4,
**caractérisée en ce qu**
on met en oeuvre des alcools gras de formule (IV)
**R**^{**8**}**OH (IV)**
dans laquelle R⁸ représente un reste alkyle et/ou alkényle linéaire ou ramifié ayant de 6 à 22 atomes de carbone.

6. Utilisation selon au moins l'une des revendications 1 à 5,
**caractérisée en ce qu'**
on met en oeuvre des éthers d'alcool gras et de polyglycol de formule (V)
**R**^{**9**}**O(CH**_{**2**}**CH**_{**2**}**O)**_{**n**}**H (V)**
dans laquelle R⁹ représente un reste alkyle et/ou alkényle linéaire ou ramifié ayant de 6 à 22 atomes de carbone et n représente des nombres allant de 1 à 20.

7. Utilisation selon au moins l'une des revendications 1 à 6,
**caractérisée en ce qu'**
on met en oeuvre des mélanges d'esterquats et d'alcools gras qu'on obtient par voie directe par alkylation d'esters d'acide gras et d'alkanolamines, dans des mélanges d'alcools gras et d'éthers d'alcool gras et de polyglycol.

8. Utilisation selon au moins l'une des revendications 1 à 7,
**caractérisée en ce qu'**
on met en oeuvre
a) de 0,1 à 25 % en poids d'esterquats,
b) de 0,5 à 90 % en poids de composés huileux,
c) de 0, 1 à 75 % en poids d'alcools gras,
d) de 0,1 à 75 % en poids d'éther d'alcool gras et de polyglycol
avec la précision que les données en quantités se complètent avec de l'eau et éventuellement avec d'autres ingrédients à 100 % en poids.

9. Utilisation selon au moins une des revendications 1 à 8,
**caractérisée en ce qu'**
on met en oeuvre les esterquats d'une part et les mélanges d'alcools gras et d'éther d'alcool gras et de polyglycol d'autre part, dans un rapport pondéral de 10 : 90 à 90 : 10.
